# EUROPEAN PATENT APPLICATION

(11) **EP 4 710 996 A2**
(43) Date of publication of application: **18.03.2026**
(21) Application number: 26155147.7
(22) Date of filing: 27.11.2024
(51) Int. Cl.: A61N 1/375

(54) **BIOSTIMULATOR HAVING EXPANDABLE FRAME**

(30) Priority: 30.11.2023 US 202363604832 P; 22.11.2024 US 202418957567
(62) Divisional of application: 24215711.3
(71) Applicant: Pacesetter, Inc., Sylmar, CA 91342 (US)
(72) Inventor: WISE, Derek, Sylmar, CA 91342 (US); ARNAR, Bernhard, Sylmar, CA 91342 (US); SERVI, Andy, Sylmar, CA 91342 (US)
(74) Representative: Barker Brettell Sweden AB

(57) **Abstract**

A biostimulator (200) includes a housing (302), an electrode extension (206), and an expandable frame (320). The housing (302) has a longitudinal axis (304) and an electronics compartment (306) containing pacing circuitry. The electrode extension (206) extends distally between the housing (302) and an electrode (212). The biostimulator (200) includes an expandable frame (320) including several struts (322) disposed about the longitudinal axis (304). Other embodiments are also described and claimed.

## Description

### TECHNICAL FIELD

The present disclosure relates to biostimulators and related biostimulator transport systems. More specifically, the present disclosure relates to leadless biostimulators and related biostimulator transport systems useful for pacing cardiac tissue.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at an intracardiac pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch (LBB) is an alternative to His-bundle pacing. Pacing at the LBB involves pacing past the His-bundle toward the right ventricular apex. More particularly, a pacing site for LBB pacing is typically below the His-bundle, on the interventricular septal wall. To achieve optimal results, the pacing site for physiological pacing at the LBB can be high on the interventricular septal wall, in the region close to the tricuspid valve and pulmonary artery outflow tract. Furthermore, the pacing site may be at a depth of up to 1.5 cm within the septal wall.

### SUMMARY

Existing leadless pacemakers may not fit, or may interfere with heart structures, when placed at an intracardiac pacing site (within the heart) for left bundle branch (LBB) pacing. More particularly, existing leadless pacemakers have bodies that are long and rigid and, when implanted at the interventricular septal wall, could extend into contact with the cardiac tissue of a ventricular free wall or the tricuspid valve during contraction of the heart. The long and rigid body of existing leadless pacemakers could also become tangled within chordae tendinae. Furthermore, a proximal end of the existing leadless pacemakers may flail within the heart chamber as the heart beats, causing cyclical contact with adjacent heart structures. Such contact could interfere with heart function. Thus, there is a need for a leadless biostimulator that can be engaged to the interventricular septal wall to pace the LBB without interfering with adjacent structures of the heart.

The present invention is defined in the independent claim. Further embodiments of the invention are defined in the dependent claims.

A biostimulator is described. In an embodiment, the biostimulator includes a housing, an electrode extension, and an expandable frame. The housing has a longitudinal axis and an electronics compartment containing pacing circuitry. The electrode extension extends distally between the housing and an electrode. The expandable frame includes several struts disposed about the longitudinal axis. The struts can expand radially outward to anchor the housing within an inferior vena cava while the electrode is implanted at a target tissue within a heart chamber. The pacing circuitry can deliver a pacing impulse through the electrode of the electrode extension to the target tissue.

A biostimulator system is described. In an embodiment, the biostimulator system includes a biostimulator transport system. The biostimulator can be mounted on the biostimulator transport system to carry the biostimulator to or from the target anatomy. A method of pacing using the biostimulator and/or the biostimulator system is also described.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic view of a patient vasculature, in accordance with an embodiment.
FIG. 2 is a diagrammatic cross section of a patient heart illustrating an example extracardiac implantation of a biostimulator having an expandable frame and an electrode extension to engage a target anatomy, in accordance with an embodiment.
FIG. 3 is a perspective view of a biostimulator having an expandable frame, in accordance with an embodiment.
FIG. 4 is a perspective view of a biostimulator having an expandable frame, in accordance with an embodiment.
FIG. 5 is a perspective view of a biostimulator system, in accordance with an embodiment.
FIG. 6 is a cross-sectional view, taken about line A-A of FIG. 5, of a biostimulator system, in accordance with an embodiment.
FIG. 7 is a flowchart of a method of pacing a target tissue using a biostimulator having an expandable frame, in accordance with an embodiment.
FIG. 8 is a perspective view of a biostimulator having an expandable frame, in accordance with an embodiment.
FIG. 9 is a flowchart of a method of pacing a target tissue using a biostimulator having an expandable frame, in accordance with an embodiment.
FIG. 10 is a diagrammatic cross section of a patient heart illustrating an example implantation of a biostimulator having an expandable frame and an integrated electrode, in accordance with an embodiment.
FIG. 11 is a diagrammatic cross section of a patient heart illustrating an example implantation of a biostimulator having an expandable frame and an integrated electrode, in accordance with an embodiment.

### DETAILED DESCRIPTION

Embodiments describe a biostimulator and a biostimulator system for pacing target tissue, e.g., septal pacing. The biostimulator may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator transport system to a specific configuration described in the various embodiments below.

In an aspect, a biostimulator includes an expandable frame to anchor a housing of the biostimulator at an extracardiac location (outside of the heart), e.g., in an inferior vena cava. The biostimulator can include an electrode extension that extends distally from the housing into a heart chamber. More particularly, an electrode of the electrode extension can engage a target tissue within the heart chamber, e.g., in a septal wall. Accordingly, when the electrode is positioned at the septal wall to deliver pacing impulses to the target tissue, the housing can be located outside of the heart to avoid interfering with a heart valve, a free heart wall opposite to the septal wall, or other heart structures. Furthermore, locating the housing outside of the heart can avoid flailing of the biostimulator within the heart, thereby reducing a likelihood of fatigue failure of the biostimulator within the heart chamber or cyclical stress on tissue at the implant site. The biostimulator therefore accommodates the limited space of a target heart chamber, and provides long-term implant stability and reduced risk of tissue trauma, particularly for patients having small heart chambers within which existing leadless pacemakers would not fit well.

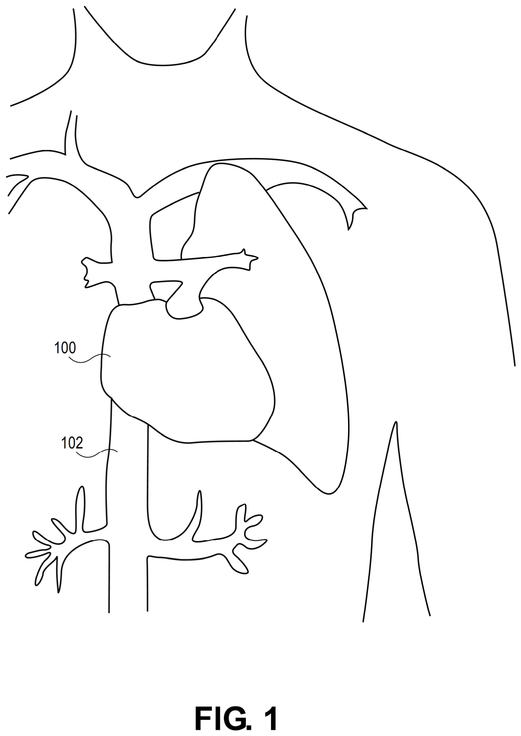

Referring to FIG. 1, a diagrammatic view of a patient vasculature is shown in accordance with an embodiment. The patient vasculature includes a heart 100 connected to numerous vascular structures, including an inferior vena cava 102. The inferior vena cava 102 carries deoxygenated blood into a right atrium of the heart 100. The deoxygenated blood subsequently flows into a right ventricle of the heart 100. Accordingly, the inferior vena cava 102 can provide access to heart chambers of the heart 100.

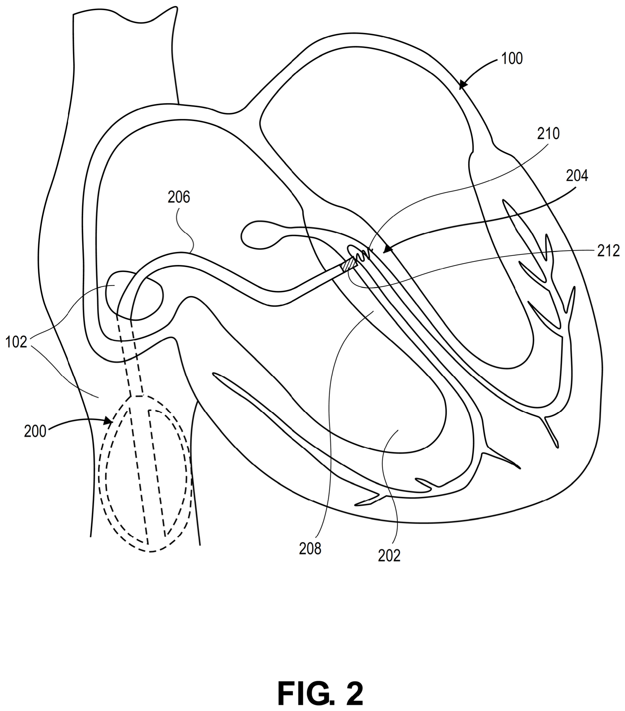

Referring to FIG. 2, a diagrammatic cross section of a patient heart illustrating an example extracardiac implantation of a biostimulator having an electrode extension to engage a target anatomy is shown in accordance with an embodiment. A biostimulator system, e.g., a cardiac pacing system, includes one or more biostimulators 200. The biostimulator 200 can be implanted in a patient anatomy, and can be leadless (and thus, may be a leadless cardiac pacemaker). A portion of the biostimulator 200 can be placed in an extracardiac anatomy, such as an upper region of the inferior vena cava 102. By contrast, a portion of the biostimulator 200 can be placed in an intracardiac anatomy, such as a heart chamber 202 (the right atrium and/or right ventricle of the heart 100). The portion of the biostimulator 200 located in the extracardiac anatomy may be positioned in the inferior vena cava 102 above a level of the renal veins. For example, the extracardiac portion of the biostimulator 200 may be implanted within 50 mm, e.g., less than 25 mm, of an entrance to the right atrium at an ostium of the inferior vena cava 102.

In an embodiment, an intracardiac portion of the biostimulator 200 can extend into and be attached to a target tissue 204 surrounding the cardiac chamber. For example, an electrode extension 206 of the biostimulator 200 can be attached to a septal wall 208 of the heart 100. More particularly, the biostimulator 200 can be delivered to the septum, and one or more elements, such as a fixation element 210 and/or an electrode 212 can pierce and advance into the septal wall 208. The electrode extension 206 can engage and electrically connect the biostimulator 200 to the target anatomy, e.g., a bundle branch in the septal wall 208.

As described below, the biostimulator 200 can have several electrode extensions having respective electrodes. The electrodes can perform deep septal pacing at respective target anatomies, such as respective heart chamber walls, bundle branches, etc. Pacing may be performed while a body of the biostimulator 200 is anchored within the extracardiac anatomy, making delivery of the electrodes to the target anatomy easier and reducing a likelihood of interaction between the biostimulator 200 and heart chamber structures.

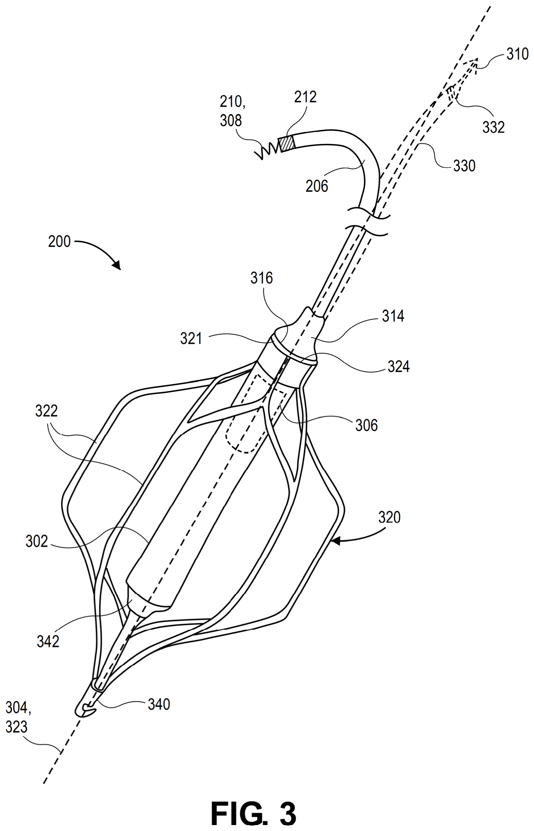

Referring to FIG. 3, a perspective view of a biostimulator is shown in accordance with an embodiment. The biostimulator 200 can be a leadless cardiac pacemaker that can perform cardiac pacing and that has many of the advantages of conventional cardiac pacemakers while extending performance, functionality, and operating characteristics. The biostimulator 200 can have one or more electrodes. For example, an electrode ring 212 on the electrode extension 206 and/or a portion of the fixation element 210 can act as an active electrode. The electrode(s) can deliver pacing pulses to the target tissue 204, and optionally, can sense electrical activity from the tissue. The electrodes may also communicate bidirectionally with at least one other device within or outside the body.

In an embodiment, the biostimulator 200 includes a housing 302 having a longitudinal axis 304. The housing 302 can contain a primary battery to provide power for pacing, sensing, and communication, which may include, for example, bidirectional communication. The housing 302 can optionally contain an electronics compartment 306 (shown by hidden lines) to hold pacing circuitry adapted for different functionality. For example, pacing circuitry in the electronics compartment 306 can include circuits for sensing cardiac activity from the electrode(s), circuits for receiving information from at least one other device via the electrode(s), circuits for generating pacing pulses for delivery to tissue via the electrode(s), or other circuitry. The electronics compartment 306 may contain circuits for transmitting information to at least one other device via the electrode(s) and can optionally contain circuits for monitoring device health. The circuit of the biostimulator 200 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

Leadless pacemakers or other leadless biostimulators 200 can be fixed to an intracardial implant site, e.g., at the septal wall 208, by one or more actively engaging mechanisms or fixation mechanisms. More particularly, the fixation element 210 can be located at a distal end of the electrode extension 206. The fixation element 210 can include a structure to engage and affix to tissue. For example, the fixation element 210 may include a helical tip 308. Alternatively, the fixation element 210 can include a non-helical fixation mechanism, such as a barb tip 310 (denoted as optional by dotted lines). Helical fixation mechanisms are shown in the figures and may include a spiral-wound wire to thread into tissue. Non-helical fixation mechanisms are represented as being portions of an optional secondary electrode extension in the figures and can include one or more barbs to pierce and grip tissue. Other fixation mechanisms include tines, hooks, etc. More particularly, the illustrated fixation mechanisms are not limiting and other fixation mechanisms, such as a freely rotating helix may be used. Furthermore, alterations to the illustrated fixation mechanisms are contemplated. For example, the barb tip 301 may include several barbs extending distally in a pitchfork configuration, and each of the barbs may have a respective electrode. Accordingly, pacing algorithms may be implemented to select different pacing vectors or select an optimal electrode from the several electrodes to stimulate the cardiac conduction system.

The electrode extension 206 can extend distally between the housing 302 and the electrode 212. For example, the biostimulator 200 can include a header assembly 314 mounted on a distal housing end 316 of the housing 302, and the electrode extension 206 can connect to and extend distally from the header assembly 314. In an embodiment, the header assembly 314 includes an electrical feedthrough. The electrode extension 206 can electrically connect to the electrical feedthrough, which passes electrical signals between the electrode 212 and the pacing circuitry within the electronics compartment 306. Accordingly, pacing impulses can be delivered through the header assembly 314 and a conductor within the electrode extension 206 to the electrode 212.

The electrode extension 206 can differ from a tradition lead at least in that the electrode extension 206 is sized to span a distance from the septal wall 208 to the location within the inferior vena cava 102, rather than to a more remote location. The electrode extension 206 is not a lead, therefore, and the biostimulator 200 is a leadless biostimulator 200. In an embodiment, the electrode extension 206 has a length of less than 40 cm, e.g., less than 25 cm. The electrode extension 206 can have an insulative jacket surrounding the electrical conductor, and the jacket may be flexible. Accordingly, the electrode extension 206 can track from a proximal extension end within the inferior vena cava 102 to a distal extension end within the septal wall 208, an atrial wall, or another target tissue surrounding one of the heart chambers 202. The electrode extension 206 can transmit the pacing impulse from the pacing circuitry to the pacing site.

Electrode placement along the electrode extension 206 can be selected to pace a particular anatomy. The electrode 212 shown in FIG. 3 is at a distal end of the electrode extension 206, immediately adjacent to the helical tip 308, and thus can pierce the target tissue 204 that is engaged by the helical tip 308. For example, the helical tip 308 can engage the left bundle branch and the electrode 212 can deliver the pacing impulse to the left bundle branch.

Alternatively, the electrode 212 (or an additional electrode) may be longitudinally offset from the distal extension end. For example, the electrode 212 may be located proximal to the distal extension end and may be spaced apart from the helical tip 308 by a distance equal to a distance between the left bundle branch and the right bundle branch. Accordingly, when the helical tip 308 engages the left bundle branch, the electrode 212 can be adjacent to the right bundle branch. The electrode 212 may therefore deliver the pacing impulse to the right bundle branch instead of, or in addition to, pacing of the left bundle branch. Electrode 212 placement may be similarly selected to pace target anatomies.

In an embodiment, the biostimulator 200 includes an expandable frame 320 to anchor the housing 302 within an extracardiac anatomy, e.g., a vessel. The expandable frame 320 can have a design, shape, and size to allow the frame to fixate to an intima of the inferior vena cava 102. For example, the expandable frame 320 can be deployed within the inferior cava to apply an outward pressure against an internal wall of the inferior vena cava 102. Optionally, as described below, the expandable frame 320 can include one or more fixation mechanisms, such as fixation hooks, to engage the intima. Accordingly, the expandable frame 320 can expand within and against the internal wall to secure the housing 302 within the inferior vena cava 102.

The housing 302 may be at least partly within the expandable frame 320. For example, the expandable frame 320 can define an internal volume within which the housing 302 is located. In an embodiment, the expandable frame 320 has a frame axis 323 that is coaxial with the longitudinal axis 304 of the housing 302. Accordingly, when the expandable frame 320 is deployed against the intima of the inferior vena cava 102 and the housing 302 is radially within the expandable frame 320, the housing 302 can be centrally located within the vessel lumen.

The expandable frame 320 can include expandable members that move from a collapsed state to an expanded state. In an embodiment, the expandable frame 320 includes several struts 322 disposed about the longitudinal axis 304. The struts 322 may be arranged symmetrically about the longitudinal axis 304 and/or the frame axis 323. For example, the struts 322 can be portions of a stent-like structure that self-expands from the collapsed state, when constrained within a sheath, to the expanded state, when deployed into the vessel lumen.

In an embodiment, the expandable frame 320 is coupled to the header assembly 314. For example, one or more of the struts 322 can be mounted on a side of the housing 302 or fixed relative to the header assembly 314. The expandable frame 320 can have a distal frame end 324 that can be at or distal to distal ends of the struts 322.

The distal frame end 324 of the expandable frame 320 can be at or proximal to the header assembly 314. For example, the expandable frame 320 may have a collar 321 that the struts 322 radiate from. The collar 321 can be mounted on the housing 302, proximal to the header assembly 314 to locate and support the struts 322 relative to the housing 302. Alternatively, the collar 321 may be mounted on the header assembly 314 having the electrical feedthrough to locate and support the struts 322 relative to the header assembly 314. Placement of the struts 322 at or proximal to the header assembly 314 can eliminate a need to pass the electrode extension 206 between the struts 322. More particularly, the struts 322 can extend proximally from the header assembly 314 and the electrode extension 206 can extend distally from the header assembly 314. Accordingly, the struts 322 and electrode extension 206 may not interfere with each other after deployment.

In addition to the electrode extension 206, the biostimulator 200 can include a second electrode extension 330. The second electrode extension 330, like the electrode extension 206, can extend from the distal housing end 316 and/or header assembly 314. For example, the second electrode extension 330 can extend distally between the housing 302 and a second electrode 332. The second electrode extension 330 may be used to deliver pacing impulses to a second target tissue, spaced apart from the target tissue engaged by the electrode extension 206. For example, the electrode extension 206 may be used to engage the left bundle branch and the second electrode extension 330 may be used to engage the right bundle branch, the right atrium, etc.

The second electrode extension 330 can have a fixation element 210 suited to a respective delivery mode and/or target anatomy. For example, delivery of a distal end of the second electrode extension 330 may be through axial loading rather than rotation. The second electrode extension 330 can include a barb tip 310 to facilitate such delivery. The barb tip 310 can have prongs that slant forward to a point. Accordingly, the barb tip 310 can be pressed into the target tissue 204 to engage the tissue, and the prongs can resist backing out of the tissue. The second electrode 332 may therefore be retained within the target tissue 204 for pacing.

In an embodiment, the biostimulator 200 includes a retrieval feature 340. The retrieval feature 340 can be mounted on a proximal housing end 342 of the housing 302. More particularly, the retrieval feature 340 can be mounted on an opposite end of the housing 302 from the electrode extension 206, which can instead be coupled to the distal housing end 316 of the housing 302. The retrieval feature 340 can facilitate precise delivery or retrieval of the biostimulator 200. For example, the retrieval feature 340 can be formed from a rigid material to allow a biostimulator transport system to engage the retrieval feature 340. The biostimulator transport system can therefore deliver the biostimulator 200 to the target tissue 204 and transmit torque to the retrieval feature 340 to screw the fixation element 210 into the target tissue 204.

The retrieval feature 340 may include a shaft extending proximally from the proximal housing end 342. A hook, bulb, or other graspable feature can be located at a proximal end of the shaft. The hook can allow a lasso, snare, or other retrieval mechanism to grasp the proximal end of the biostimulator 200. The retrieval mechanism can be pulled to retract the biostimulator 200 from the patient anatomy.

In an embodiment, the struts 322 connect to the shaft of the retrieval feature 340. For example, the struts 322 can terminate along the shaft, distal to the hook. The struts 322 may therefore be supported at both a distal end, near the distal housing end 316, and a proximal end, near the proximal housing end 342. The expandable frame 320 can therefore have two closed ends, providing for a closed-frame structure.

It may be advantageous for the expandable frame 320 to change in length, e.g., in an axial or longitudinal direction, in order to transition between the expanded state and the collapsed state. For example, the expandable frame 320 may be longer when it is in the collapsed state, e.g., while residing inside the biostimulator transport system 504, than when it is in the expanded state, e.g., while deployed or implanted in the anatomy. A change in length of the expandable frame 320 may be provided by relative movement between a proximal end of the expandable frame 320 and the housing 302. For example, the expandable frame 320 may slide over the shaft extending from the proximal housing end 342, allowing the struts 322 to lengthen during collapse or shorten during expansion. Alternatively, the retrieval feature 340 may be directly coupled to a proximal end of the expandable frame 320, and a longitudinal gap may be present between the proximal housing end 342 and the retrieval feature 340. The struts 322 can accordingly lengthen during collapse or shorten during expansion of the frame, with the housing 302 freely supported in a cantilever fashion from the distal housing end 316.

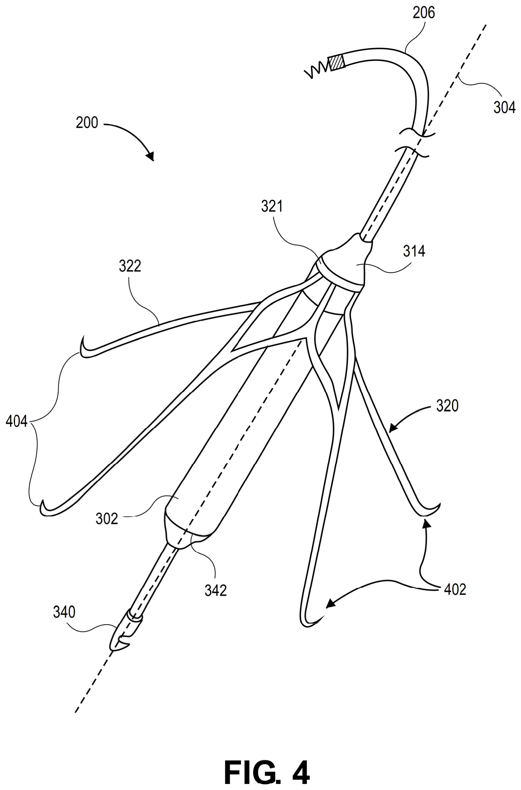

Referring to FIG. 4, a perspective view of a biostimulator is shown in accordance with an embodiment. The expandable frame 320 may have an open-frame structure. In an embodiment, the struts 322 have respective free ends 402. More particularly, when the struts 322 expand radially outward into the expanded state, the struts 322 can extend from the housing 302, e.g., at the collar 321, to the free ends 402 that are radially outward of the housing 302. The struts 322 can extend along a strut axis that is slanted relative to the longitudinal axis 304. The slant may be in a proximal direction from the location near the header assembly 314 to the free ends 402 that are radially outward of the housing 302.

The struts 322 can include anchoring mechanisms to attach the expandable frame 320 to the intima of the inferior vena cava 102. In an embodiment, the struts 322 include respective fixation hooks 404. The fixation hooks can be at the free ends 402, which may be located further outward of the longitudinal axis 304 than any other location on the expandable frame 320. Accordingly, when the expandable frame 320 radially expands within the inferior vena cava 102, the hooks can engage the intima and secure the housing 302 within the vessel lumen.

The expandable frame 320 is described above as surrounding the housing 302 of the biostimulator. It will be appreciated, however, that the expandable frame 320 may be located distal or proximal to the housing 302. For example, the expandable frame 320 may be connected to and extend proximally from the proximal housing end 342. More particularly, the expandable frame 320 can be an open- or closed-frame structure that is longitudinally offset (distal to or proximal to) the housing 302. When located proximal to the housing 302, the frame can expand to appose the intima of the inferior vena cava 102 and the housing 302 and electrode extension 206 can be secured in the vessel lumen distal to the expandable frame 320.

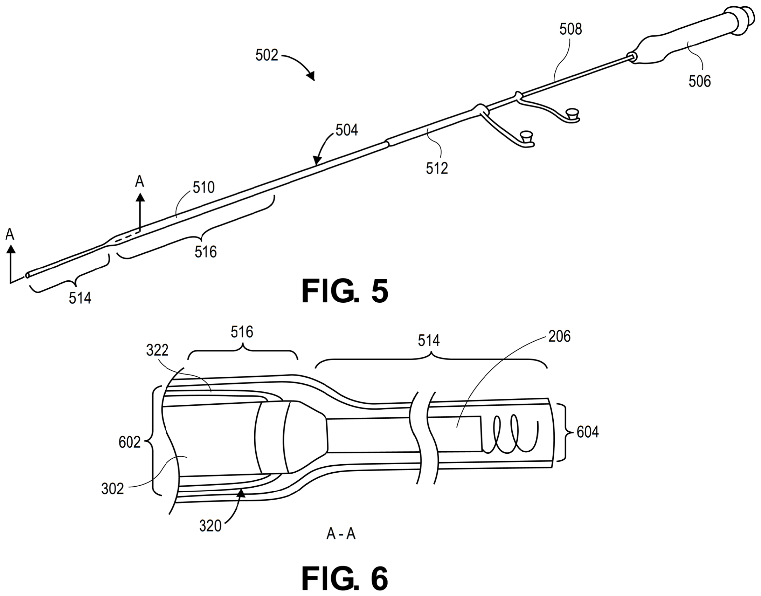

Referring to FIG. 5, a perspective view of a biostimulator system is shown in accordance with an embodiment. A biostimulator system 502 can include the biostimulator 200, e.g., a leadless pacemaker or other leadless biostimulator. The biostimulator system 502 can include delivery or retrieval systems, which may be catheter-based systems used to carry the biostimulator 200 intravenously to or from a patient anatomy. For example, a biostimulator transport system 504 can be used to deliver the biostimulator to, or retrieve the biostimulator from, a patient. The biostimulator 200 can be attached, connected to, or otherwise mounted on the biostimulator transport system 504. The biostimulator 200 can be mounted on and/or within a distal end of a catheter of the biostimulator transport system 504. The biostimulator 200 can thereby be advanced intravenously into or out of the heart 100.

The biostimulator transport system 504 can include a handle 506 to control movement and operations of the transport system from outside of a patient anatomy. One or more elongated members extend distally from the handle 506. For example, an elongated catheter body 508 can extend distally from the handle 506. The elongated catheter body 508 can extend to a distal end of the biostimulator transport system 504. In an embodiment, the biostimulator 200 is mounted on a distal end of the elongated catheter body 508.

The biostimulator transport system 504 can include a protective sleeve 510 to cover the biostimulator 200 during delivery and implantation. The protective sleeve 510 can extend over, and be longitudinally movable relative to, the elongated catheter body 508. The biostimulator transport system 504 may also include an introducer sheath 512 that can extend over, and be longitudinally movable relative to, the protective sleeve 510. The introducer sheath 512 can cover a distal end of the protective sleeve 510, the elongated catheter body 508, and the biostimulator 200 as those components are passed through an access device into the patient anatomy.

Several components of the biostimulator transport system 504 are described above by way of example. It will be appreciated, however, that the biostimulator transport system 504 may be configured to include additional or alternative components. More particularly, the biostimulator transport system 504 may be configured to deliver and/or retrieve the biostimulator 200 to or from the target anatomy. Delivery and/or retrieval of the biostimulator 200 can include retaining the biostimulator 200 during transport to the target anatomy and rotational or axial loading of the biostimulator 200 during implantation of the biostimulator 200 at the target anatomy. Accordingly, the biostimulator transport system 504 can incorporate features to retain, rotate, push, or pull the biostimulator 200.

The biostimulator 200 can be carried at a distal end of the biostimulator transport system 504. For example, the biostimulator transport system 504 can include a grip mechanism to connect to the retrieval feature 340 of the biostimulator 200. The grip mechanism may engage the retrieval feature 340 internally or externally.

The biostimulator transport system 504 can include a distal section 514 and a proximal section 516. The proximal section 516 can include a section of the protective sleeve 510 that contains the housing 302 of the biostimulator 200 during delivery. The proximal section 516 can be proximal to the distal section 514, which can include a section of the protective sleeve 510 that contains the electrode extension 206 during delivery. As described below, the sections can be sized to fit and conform to the respective biostimulator portions.

Referring to FIG. 6, a cross-sectional view, taken about line A-A of FIG. 5, of a biostimulator system is shown in accordance with an embodiment. The body of the biostimulator 200 includes the housing 302 and the struts 322 of the expandable frame 320 surrounding the housing 302. The electrode extension 206 can include the narrow extension cable that extends distally from the housing 302. The proximal section 516, which covers the body of the biostimulator 200, may therefore be larger than the distal section 514, which covers the electrode extension 206. More particularly, the proximal section 516 can have a proximal inner dimension 602 that is at least as large as the profile of the proximal portion of the biostimulator body, and the distal section 514 can have a distal inner dimension 604 that is as large as the profile of the electrode extension 206. The proximal inner dimension 602 can therefore be larger than the distal inner dimension 604. More particularly, the proximal section 516 can have a larger inner dimension than the distal section 514.

In an embodiment, the distal section 514 can be elastic to allow it to expand when retracted over the housing 302. The elastic length of the distal section 514 may be formed from an elastomeric material. For example, the distal section 514 may be formed from a urethane tubing. Accordingly, the elastic distal section 514 can conform to the portion of the biostimulator 200 that it covers. In a delivery state, as shown in FIG. 6, the distal inner dimension 604 may have a same size as the electrode extension 206. When the protective sleeve 510 is retracted and the distal section 514 is pulled over the housing 302 to expose the electrode extension 206, the distal inner dimension 604 can have a same profile as the housing 302 and the expandable frame 320.

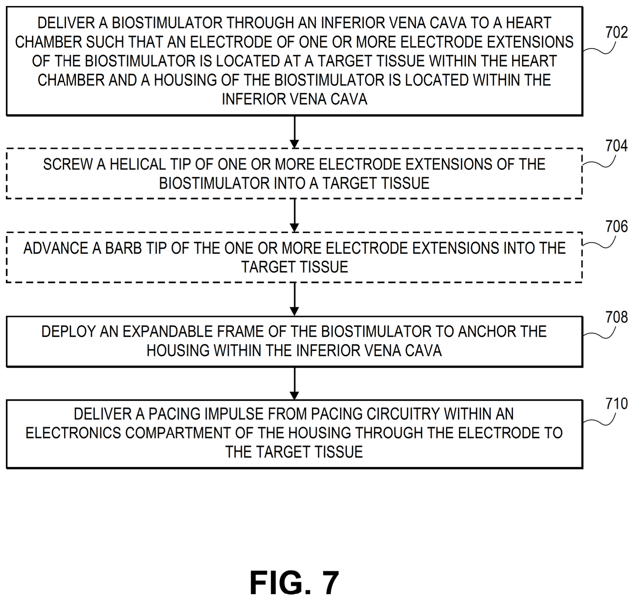

Referring to FIG. 7, a flowchart of a method of pacing a target tissue using a biostimulator is shown in accordance with an embodiment. At operation 702, the biostimulator 200 is delivered through the inferior vena cava 102 to the heart chamber 202. Delivery can include advancement of the distal section 514 of the biostimulator transport system 504 through the inferior vena cava 102 into the target chamber. The distal end of the distal section 514 can be located at the target tissue 204 within the heart chamber 202. When the distal section 514 is adjacent to the target tissue 204, the electrode extension 206 of the biostimulator 200 may similarly be located at the target tissue 204 within the heart chamber 202. By contrast, the proximal section 516 of the protective sleeve 510, and the housing 302 of the biostimulator 200, may be located at an extracardiac location, e.g., within the inferior vena cava 102.

One or more electrode extensions of the biostimulator 200 can be delivered into the target tissue 204. For example, optionally at operation 704, the helical tip 308 of the electrode extension 206 can be screwed into the target tissue 204. Screwing the helical tip 308 into the target tissue 204 may be achieved by retracting the protective sleeve 510 to expose a portion of the electrode extension 206 including the helical tip 308 and then rotating the transport system at the handle 506. The rotation can be transmitted through the elongated catheter to the biostimulator 200 to screw the helical tip 308 into the target tissue 204.

One or more of the electrode extensions may be advanced into the target tissue 204 axially. More particularly, one or more of the electrode extensions can be pushed into the target tissue 204. Whereas the electrode extension 206 may be twisted into the target tissue 204 at a first anatomical location, another electrode extension, e.g., the second electrode extension 330, may be advanced into the target tissue 204 at a separate location. For example, when the protective sleeve 510 is retracted, a stylet or another pushing member may be used to plunge the barb tip 310 of the second electrode extension 330 into a second target tissue 204, such as within the right atrium. It will be appreciated that additional delivery system features may be incorporated in the biostimulator transport system 504 to control the engagement of one or more electrode extensions with respective target tissues 204. Accordingly, the electrode extensions can be engaged to the target sites to implant the electrodes for pacing.

In the configuration described above, the biostimulator 200 can have a single electrode extension which may be implanted in the right ventricle to pace a corresponding target tissue 204, such as the left bundle branch. The above description also refers to the configuration having two electrode extensions, which can be placed in an atrial and ventricular location to enable dual chamber atrio-ventricular (AV) pacing. It will be appreciated that the biostimulator 200 may have more than two electrode extensions. For example, the biostimulator 200 may have three electrode extensions. The use of three electrode extensions can enable cardiac resynchronization therapy pacing (CRT-P). More particularly, the left ventricle can be stimulated by one electrode extension placed in the coronary sinus ostium. Another electrode extension may pace the His bundle, and the third electrode extension can pace the left bundle branch. Accordingly, the number and placement of electrode extensions may be selected to achieve the desired therapeutic effect.

At operation 708, the expandable frame 320 of the biostimulator 200 is deployed to anchor the housing 302 within the inferior vena cava 102. The protective sleeve 510 may be retracted further to pull the distal section 514 over the housing 302 and the expandable frame 320. When the distal section 514 is proximal to the expandable frame 320, the expandable frame can radially expand. The frame can press outward against the intima of the inferior vena cava 102 to secure and anchor the biostimulator 200 within the vessel lumen. The biostimulator transport system 504 can be retracted away from the biostimulator 200, e.g., by releasing the retrieval feature 340. The biostimulator 200 can therefore remain implanted within the patient anatomy while biostimulator transport system 504 can be removed therefrom.

At operation 710, pacing impulses are delivered from the pacing circuitry within the electronic compartment of the housing 302. The pacing impulse can be transmitted through the header assembly 314 and the conductor of the electrode extension 206 to the electrode 212. The pacing impulse can pass through the electrode 212 to the target tissue 204. Biostimulator 200 can therefore deliver therapeutic pacing to target tissue 204.

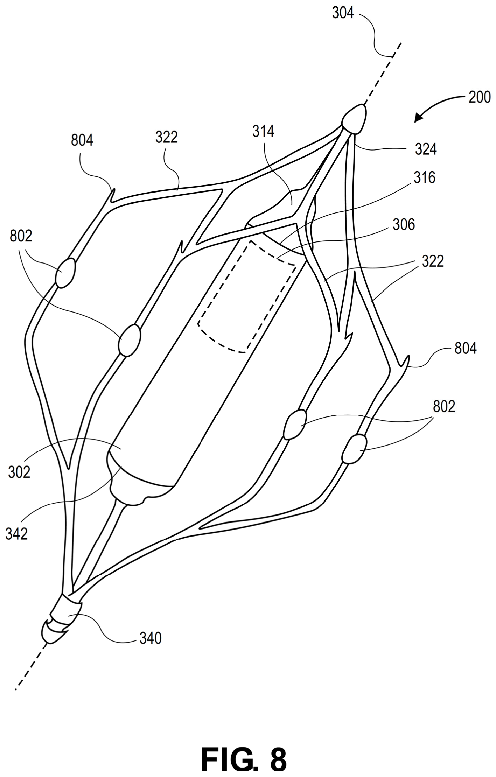

Referring to FIG. 8, a perspective view of a biostimulator having an expandable frame is shown in accordance with an embodiment. Pacing of the right ventricle and/or septum can require that a pacing lead, e.g., the electrode extension, cross a tricuspid valve. The lead or extension can therefore transmit or carry pacing signals across the tricuspid valve from the pulse generator to the pacing site. Such crossing of the tricuspid valve may, however, require additional operations to affix the lead or extension to the target tissue before or after implanting the housing in the inferior vena cava. In addition to the procedural challenges that such operations may create, the lead or extension has a potential to increase a risk of valve damage or other complications when residing permanently across the tricuspid valve between the housing and the fixation element.

In an embodiment, a biostimulator 200 and/or biostimulator system 502 includes a biostimulator configured to be deployed in a heart chamber, downstream of the inferior vena cava. The downstream deployment location can avoid requiring an extension bridging the tricuspid valve after implantation and, thus, can reduce a likelihood of valve damage. As described below, the biostimulator can be implanted in an outflow tract of the heart chamber, e.g., a right ventricular outflow tract (RVOT) or in a chamber appendage, e.g., a right atrial appendage (RAA). In any case, the expandable frame of the biostimulator can include integrated electrode features that deliver pacing impulses directly to the target tissue at the site of implantation, rather than via a lead or extension.

In an embodiment, the biostimulator 200 can have one or more strut electrodes 802. For example, the expandable frame 320 can include several struts 322, as described above, and the one or more strut electrodes 802 may be mounted on respective struts 322. The strut electrodes 802 can act as an active electrodes. More particularly, the strut electrode(s) 802 can deliver pacing pulses to the target tissue 204, and optionally, can sense electrical activity from the tissue. The electrodes may also communicate bidirectionally with at least one other device within or outside the body.

In an embodiment, the biostimulator 200 includes the housing 302 having a longitudinal axis 304. The housing 302 can contain a primary battery to provide power for pacing, sensing, and communication, which may include, for example, bidirectional communication. The housing 302 can optionally contain an electronics compartment 306 (shown by hidden lines) to hold pacing circuitry adapted for different functionality. For example, pacing circuitry in the electronics compartment 306 can include circuits for sensing cardiac activity from the pacing electrode(s), circuits for receiving information from at least one other device via the electrode(s), circuits for generating pacing pulses for delivery to tissue via the strut electrode(s) 802, or other circuitry. The electronics compartment 306 may contain circuits for transmitting information to at least one other device via the strut electrode(s) 802 and can optionally contain circuits for monitoring device health. The pacing circuitry can conduct signals to the strut electrode(s) 802 directly via the struts 322, or through electrical leads running over the struts. The struts or electrical leads may be insulated to prevent shorting of the signal to the target tissue through any portion of the biostimulator 200 other than the strut electrode(s) 802.

Leadless pacemakers or other leadless biostimulators 200 can be fixed to an intracardial implant site, e.g., at a RVOT or RAA, by one or more actively engaging mechanisms or fixation mechanisms. For example, the fixation element can include one or more tines 804 extending outward from the struts 322 of the expandable frame 320. In an embodiment, the tines 804 are outwardly directed to engage the target tissue 204 against which the expandable frame 320 is deployed. For example, the tines 804 can hook, pierce, or otherwise grip the target tissue 204 of the RVOT or RAA.

Electrode placement along the struts 322 of the expandable frame 320 can be selected to pace a particular anatomy. For example, the electrodes may be located on radially outward surfaces of the expandable frame 320 to engage the annular interior surface surrounding the RVOT or RAA. When the tines 804 anchor the expandable frame 320 in the target locations, the electrodes can be activated to perform tissue pacing. For example, the expandable frame 320 can be deployed in the RVOT or RAA and the electrodes may be activated to pace major conduction system structures in close proximity to the deployed device, such as the HIS bundle, right bundle branch, or left bundle branch.

In an embodiment, electrodes may be independently activated. For example, the pacing circuitry can deliver pacing impulses to one or more of the strut electrodes 802 while not energizing one or more of the strut electrodes 802. The strut electrodes 802 may therefore be used in conjunction with software algorithms to select different electrodes for different pacing or sensing modalities. For example, the pacing circuitry can be driven by a computer program to select a pacing modality to optimize thresholds/impedance, or to control pacing vectors. The pacing vectors, or vector patterns, can direct impulses to the target tissue 204, e.g., the conduction system within the septal wall 208, without directing energy to an orifice wall of the RVOT opposite of the septal wall 208, for example.

As described above, the expandable frame 320 can have a design, shape, and size to allow the frame to fixate to an intima of the target anatomy. For example, the expandable frame 320 can be deployed within the RVOT or RAA to apply an outward pressure against an internal wall of those anatomical structures. The expandable frame 320 can expand within and against the internal wall to secure the housing 302 within the anatomical structures.

The housing 302 may be at least partly within the expandable frame 320. For example, the expandable frame 320 can define an internal volume within which the housing 302 is located. In an embodiment, the expandable frame 320 has a frame axis 323 that is coaxial with the longitudinal axis 304 of the housing 302. Accordingly, when the expandable frame 320 is deployed against the intima of the inferior vena cava 102 and the housing 302 is radially within the expandable frame 320, the housing 302 can be centrally located within the vessel lumen.

The expandable frame 320 can include expandable members that move from a collapsed state to an expanded state. In an embodiment, the expandable frame 320 includes several struts 322 disposed about the longitudinal axis 304. The struts 322 may be arranged symmetrically about the longitudinal axis 304 and/or the frame axis 323. For example, the struts 322 can be portions of a stent-like structure that self-expands from the collapsed state, when constrained within a sheath, to the expanded state, when deployed into the vessel lumen.

In an embodiment, the expandable frame 320 is coupled to the header assembly 314. For example, one or more of the struts 322 can be mounted on a side of the housing 302 or fixed relative to the header assembly 314. The expandable frame 320 can have a distal frame end 324 that can be at or distal to distal ends of the struts 322.

In an embodiment, the biostimulator 200 includes the retrieval feature 340. The retrieval feature 340 can facilitate precise delivery or retrieval of the biostimulator 200. For example, the retrieval feature 340 can be formed from a rigid material to allow the biostimulator transport system 504 to engage the retrieval feature 340. The biostimulator transport system 504 can therefore deliver the biostimulator 200 to the target tissue 204 and manipulate or move the biostimulator 200 relative to the target tissue 204. The retrieval feature 340 may include the any of the hook, bulb, or other graspable features described above.

In an embodiment, the struts 322 connect to the shaft of the retrieval feature 340. For example, the struts 322 can terminate along the shaft, distal to the graspable feature of the retrieval feature 340. The struts 322 may therefore be supported at both a distal end, near the distal housing end 316, and a proximal end, near the proximal housing end 342. The expandable frame 320 can therefore have two closed ends, providing for a closed-frame structure. Alternatively, the expandable frame 320 may have an open-frame structure, as described above with respect to FIG. 4. In any case, the strut electrodes 802 integrated on the struts 322 of the expandable frame 320 can engage and pace the target tissue 204.

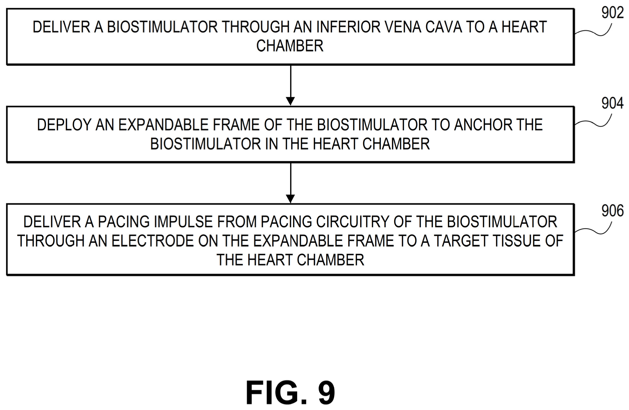

Referring to FIG. 9, a flowchart of a method of pacing a target tissue using a biostimulator having an expandable frame is shown in accordance with an embodiment. Deploying the biostimulator 200 having an expandable frame 320 with integrated electrodes in the heart chamber 202 can provide the advantages described above, including eliminating the electrode extension and/or not requiring the extension to remain in the tricuspid valve after implantation. Additionally, the system parts can be located away from the tricuspid valve and other critical structures to reduce the risk of chordae tendineae damage or free wall interaction. Furthermore, the integrated electrodes can stimulate the native cardiac conduction system, which can energize more of the conduction system and cardiac tissue.

At operation 902, the biostimulator 200 is delivered through the inferior vena cava to the heart chamber 202. For example, the biostimulator 200 can be loaded into the biostimulator 200 transport system, and the transport system may then be advanced through the vasculature, e.g., via a femoral vein access, to carry the biostimulator 200 through the tricuspid valve into the right atrium.

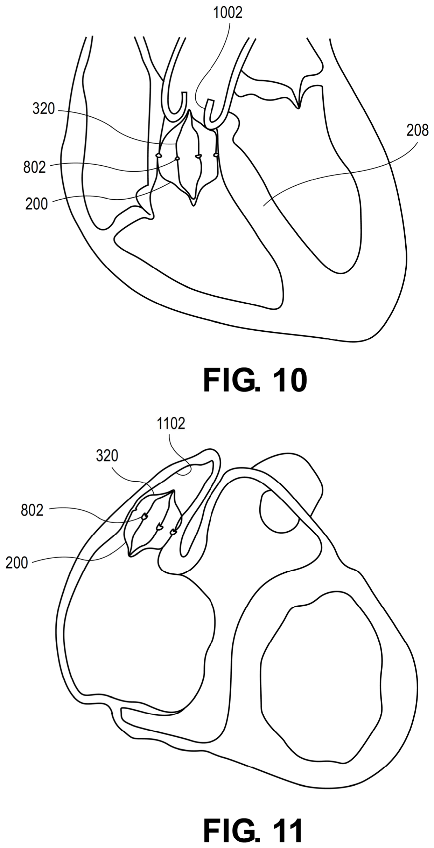

At operation 904, the expandable frame 320 of the biostimulator 200 is deployed to anchor the biostimulator 200 in the heart chamber 202. The expandable frame 320 can expand to cause the tines 804 to engage the tissue surrounding the deployment location, e.g., an orifice of the RVOT or RAA, to fixate the biostimulator 200 in the expanded state. Examples of expansion locations are shown and described with respect to FIGS. 10-11 below.

Referring to FIG. 10, a diagrammatic cross section of a patient heart illustrating an example implantation of a biostimulator 200 having an expandable frame 320 and an integrated electrode 802 is shown in accordance with an embodiment. The biostimulator 200 can expand within the RVOT. In an embodiment, the expandable frame 320 is expanded within the orifice of the RVOT against the pulmonary valve 1002. Accordingly, the strut electrodes 802 of the biostimulator 200 can expand against the surrounding tissue, including the tissue of the septal wall 208.

Referring to FIG. 11, a diagrammatic cross section of a patient heart illustrating an example implantation of a biostimulator 200 having an expandable frame 320 and an integrated electrode 802 is shown in accordance with an embodiment. The biostimulator 200 can expand within the RAA 1102. More particularly, the expandable frame 320 can expand against the interior surface of the RAA orifice. Accordingly, the strut electrodes 802 can expand against the surrounding tissue, including conduction system regions adjacent to the RAA tissue.

Whether deployed in the RVOT or RAA, the biostimulator 200 may not occlude blood passage. Blood can flow through the expandable frame 320 from the heart chamber 202 into the RAA or the pulmonary vein, for example. Accordingly, the biostimulator 200 may pace, but not occlude the target anatomies.

Referring again to FIG. 9, at operation 906, a pacing impulse is delivered from the pacing circuitry of the biostimulator 200 through the strut electrode(s) 802 on the expandable frame 320 to the target tissue 204 of the heart chamber 202. The pacing impulses may be directed through the tissue of the RAA 1102 or the RVOT to an underlying conductive system structure, such as a His bundle or a left bundle branch. The implanted biostimulator 200 may therefore provide therapy to the conduction system.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (200), comprising:
a housing (302) having a longitudinal axis (304) and an electronics compartment (306) containing pacing circuitry;
an expandable frame (320) including a plurality of struts (322) disposed about the longitudinal axis (304); and
one or more strut electrodes (802) mounted on respective struts (322) of the plurality of struts (322).

2. The biostimulator of claim 1, wherein the one or more strut electrodes (802) are configured to deliver pacing pulses to target tissue (204).

3. The biostimulator of claim 2, wherein the one or more strut electrodes (802) are independently activatable to deliver the pacing pulses.

4. The biostimulator of any one of claims 1 to 3, wherein the plurality of struts (322) are insulated.

5. The biostimulator of any one of claims 1 to 4, further comprising one or more tines (804) extending outward from the plurality of struts (322).

6. The biostimulator of any one of claims 1 to 5, wherein the one or more strut electrodes (802) are located on radially outward surfaces of the plurality of struts (322).

7. The biostimulator of any one of claims 1 to 6, wherein the expandable frame (320) defines an internal volume containing the housing (302).

8. The biostimulator of claim 7, wherein the housing (302) is centrally located within the internal volume.

9. The biostimulator of any one of claims 1 to 8, wherein the expandable frame (320) has a frame axis (323) that is coaxial with the longitudinal axis (304) of the housing (302).

10. The biostimulator of any one of claims 1 to 9, wherein the plurality of struts (322) are disposed symmetrically about the longitudinal axis (304).

11. The biostimulator of any one of claims 1 to 10, wherein the expandable frame (320) is self-expandable from a collapsed state to an expanded state.

12. The biostimulator of any one of claims 1 to 11, wherein the expandable frame (320) has a distal closed end distal to the housing (302) and a proximal closed end proximal to the housing (302).

13. The biostimulator of any one of claims 1 to 12, further comprising a retrieval feature (340) having a shaft extending proximally from a proximal end (342) of the housing (302), wherein the plurality of struts (322) terminate along the shaft.

14. A biostimulator system (502), comprising:
a biostimulator transport system (504); and
the biostimulator (200) of any of one of claims 1 to 13 mounted on the biostimulator transport system (504).

15. The biostimulator system of claims 13 and 14, wherein the biostimulator transport system (504) includes a retrieval system configured to grasp the retrieval feature (340) of the biostimulator (200).
